(19) Europäisches Patentamt

European Patent Office

Office européen des brevets

(11) **EP 3 705 033 A1**

(12) **EUROPEAN PATENT APPLICATION**

(43) Date of publication:
**09.09.2020 Bulletin 2020/37**

(51) Int Cl.:
*A61B 5/021* (2006.01)    *A61B 5/022* (2006.01)
*A61B 5/00* (2006.01)    *A61B 5/02* (2006.01)

(21) Application number: **19161194.6**

(22) Date of filing: **07.03.2019**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Koninklijke Philips N.V.
5656 AG Eindhoven (NL)**

(72) Inventors:
• **BOGATU, Laura
5656 AE Eindhoven (NL)**

• **MUEHLSTEFF, Jens
5656 AE Eindhoven (NL)**
• **BRESCH, Erik
5656 AE Eindhoven (NL)**
• **KUENEN, Maarten Petrus Joseph
5656 AE Eindhoven (NL)**
• **WOERLEE, Pierre
5656 AE Eindhoven (NL)**

(74) Representative: **Philips Intellectual Property &
Standards
High Tech Campus 5
5656 AE Eindhoven (NL)**

(54) **BLOOD PRESSURE MEASUREMENT DEVICE AND CONTROL METHOD**

(57)    A blood pressure measurement device (12) has an inflatable chamber (20) with a variable inflation level for applying a controllable pressure to a part (16) of a subject's body, and a volume varying arrangement (24) for inducing an additional change in an internal volume of the chamber (20) without altering a quantity of fluid inside the chamber. A controller (32) is configured to execute a volume change procedure using the volume varying arrangement, wherein one or more defined volume changes are induced in the chamber, with the fluid quantity kept the same. An internal pressure change response inside the chamber is simultaneously monitored. An output is then generated based on the sensed pressure changes. In some examples, a pressure versus volume relationship for the inflatable chamber (20) may be generated and output.

FIG. 1

**Description**

FIELD OF THE INVENTION

The present invention relates to a blood pressure measurement device, in particular a body-mountable blood pressure measurement device, and a method for configuring the device.

BACKGROUND OF THE INVENTION

When monitoring a patient's condition in a clinical setting, early detection of critical states of the patient is important. Common monitored parameters of a patient such as heart rate or blood pressure lack specificity in indicating a particular condition or problem, and are often encumbered by a time lag between patient deterioration and change in the parameter. For example, a hypotension phase may only become apparent from a blood pressure signal after the phase has been entered, thus precluding any preventative action.

One valuable source of clinical information, which is currently under-explored, is that of arterial (or vascular) compliance. This is indicative of various parameters related to circulatory integrity. Variations in this parameter are linked to changes in blood pressure, circadian rhythms, physical activity, stress, as well as other longer-term changes caused for instance by age or lifestyle. Arterial compliance can hence reveal a large amount of information regarding health status.

Arterial compliance corresponds to the ability of arteries (specifically artery walls) to distend and contract in response to changes in arterial pressure (i.e. pressure exerted on the artery wall by flowing blood). Arterial compliance can be defined by the expression

$$C_{art}\left(P_{tm}(t)\right) = \frac{dV_{art}(t)}{dP_{art}(t)} \qquad (1)$$

where $V_{art}$ is arterial volume, $t$ is time, $P_{art}$ is arterial blood pressure and $P_{tm}$ is transmural pressure (i.e. the net pressure across the artery wall). Transmural pressure, $P_{tm}$ is defined as the arterial blood pressure, $P_{art}$, minus any applied pressure from the outside onto the artery wall, i.e. artery internal pressure minus externally applied pressure.

Arterial blood pressure $P_{art}$ oscillates with each heart cycle, with the maximum or peak value of $P_{art}$ each cycle corresponding to systolic blood pressure (SBP) and the minimum value of $P_{art}$ each cycle corresponding to diastolic blood pressure (DBP). The SBP value is the blood pressure value at the end systole phase of the heart cycle. The DBP value is the blood pressure value at the end diastole point of the heart cycle.

Arterial compliance is affected by many different factors, and is highly person specific. It is a function in part of the transmural pressure across the artery wall ($P_{tm}$). Hence it has different values depending on the net pressure across the artery wall.

Due to its dependency on multiple physiological factors, monitoring of arterial compliance has a large number of possible applications for detecting patient deterioration.

One straightforward approach is to simply monitor changes in the arterial compliance function, and for instance present these on a display for analysis by a clinician.

Another approach is to incorporate arterial compliance information into one or more physiological models (such as that describing the Windkessel effect), for the purpose of approximating further parameters such as cardiac output or peripheral resistance (the resistance of arteries to blood flow).

In further examples, knowledge of arterial compliance can be used as a convenient (non-invasive) surrogate for blood pressure estimation.

A further example application is the use of compliance information for assessment of endothelial function (which influences the ability of the arteries to adjust lumen size to maintain homeostasis). Usually this would be performed by means of measuring artery dimensions following an induced vascular occlusion of several minutes (flow-mediated dilation). The short-term changes in blood flow produce changes in arterial lumen size, revealing information about stability of a patient (such as presence of sepsis), or progression of cardiovascular diseases. Ultrasound, photoplethysmography (PPG) or pulse arrival time (PAT) based techniques are currently employed to assess the artery dimensions.

These current techniques suffer deficiencies related to the difficulty in imaging arteries (in the case of ultrasound), and/or inconvenience for the patient, due to the fact that occlusion needs to take place for several minutes to produce a detectable change in artery size. This is uncomfortable for a patient.

Monitoring compliance may allow for more long term or ongoing assessment of endothelial response, by enabling more accurate and comfortable measurements of changes in artery size compared to current practices. In particular, duration of induced occlusion of the artery can be shortened while still achieving sufficient change in artery size for measurement of endothelial response. This allows for monitoring of endothelial response over a longer or ongoing period without causing significant discomfort for the patient.

A further example application of arterial compliance information is assessment of fluid responsiveness. Arterial compliance allows for calculating of mean systemic filling pressure for example. Mean systemic filling pressure can be derived by measurement of stop-flow forearm arterial and venous equilibrium pressures. These pressure values are input to a Guytonian model. This model allows for inferring of the mean systemic filling pressure and also the effective intravascular volume status, which contributes to the assessment of fluid responsiveness. Information about compliance permits improvement in the accuracy of the parameters used in such a model, thus leading to a better quantification of fluid responsiveness.

In a similar approach, measurement of arterial compliance could also be incorporated into assessment in limb blood flow, measured by means of venous occlusion plethysmography. This measurement is also based on employing a model which describes the physiology of the occluded limb. Accurate estimation of the model's parameters (e.g. compliance) can assist in interpreting changes in limb volume during this measurement (e.g. detection of venous thrombosis).

Hence there are many useful applications for arterial compliance.

In principle compliance can be measured by means of high resolution imaging (e.g. ultrasound), which can reveal changes in arterial diameter as arterial pressure varies between systolic and diastolic values. However, this is highly resource intensive, as it requires highly skilled operators to perform the ultrasound scan. It is also prone to errors from potential misinterpretation of the images. It also does not allow for continuous or long-term monitoring of arterial compliance, as a single scan can naturally only be representative of compliance at one point in time.

Furthermore, if information about compliance is required over a wider range of transmural pressures, then imaging technologies would need to be calibrated with steps for applying external pressure to arteries, further complicating the measurement.

An alternative approach to ultrasound imaging is to use the (typically) already available physiological signals used in clinics for patient monitoring, e.g. electrocardiogram (ECG), PPG and/or a sphygmomanometer (blood pressure measurement cuff). These signals can give information about changes in arterial volume and pressure, enabling calculation of compliance. These approaches thus allow for more practical continuous monitoring of compliance.

A sphygmomanometer is a form of blood pressure monitoring device. It comprises an inflatable cuff with an internal inflation chamber. The cuff is wrapped around a patient's arm and by inflation of the cuff, pressure is applied to the brachial artery beneath the cuff. By monitoring pressures exerted on the cuff by the artery beneath it as the applied pressure is varied, an accurate measure of arterial blood pressure can be derived.

One form of blood pressure monitoring device is an oscillometric blood pressure measurement device. This is based on detecting oscillations (i.e. oscillometric pressure signals) in the artery wall as an applied pressure is varied. These manifest in oscillations in transmural pressure across the artery wall. The amplitudes of these oscillatory signals can be used to derive an indication of systolic and diastolic blood pressure.

These oscillations are measured across a range or spectrum of different externally applied pressures. Where the device comprises an inflatable cuff, this can be achieved by gradually inflating the inflatable cuff. Another form of pressure application means however can alternatively be used. The pressure variation rate is small compared to the rate of the blood pressure oscillations. Hence over small time scales, the change in detected transmural pressure can be assumed equal to the change in arterial pressure. In this way, arterial pressure oscillations can be derived.

Where the device comprises an inflatable cuff for instance, measurement of the oscillations in transmural pressure may be by means of an internal air pressure sensor in the cuff which measures oscillations in the internal inflation chamber pressure. These are indicative of the oscillating pressure applied to the cuff by the oscillating wall of the artery.

It is also known to derive measures of arterial volume variation using blood pressure signals obtained from oscillometric blood pressure measurement devices. Prior knowledge for instance of the elasticity or compliance of the measurement device (e.g. the inflatable cuff, where this is used) allows detected pressure changes to be processed to derive the corresponding spatial displacement, and thus the corresponding arterial volume changes.

A problem with such approaches is that the response to arterial volume oscillations of the blood pressure measurement cuff varies depending upon a large number of factors. These include the tightness of the cuff wrapping around the subject's arm (or other body part, e.g. leg), the cuff air volume level, temperature of cuff air, inflation speed, arm tissue characteristics, arm size, frequency components of arterial waveform (viscoelasticity of measurement device material), time passed since previous inflation. This makes preparing in advance a reliable conversion function between sensed cuff pressure and derived arterial volume very difficult.

An improved approach for configuring an inflation-based blood pressure measurement device in a manner suitable for measuring arterial compliance would therefore be of value.

SUMMARY OF THE INVENTION

The invention is defined by the claims.

According to examples in accordance with an aspect of the invention, there is provided a blood pressure measurement device for mounting to a part of a body of a subject, for applying a variable pressure in use to said part of the body, the device comprising:

- a fluid-inflatable chamber for receiving a variable quantity of fluid for inducing said variable pressure to the part of the body in use;
- a volume varying arrangement for inducing a defined change in an internal volume of said fluid-inflatable chamber without altering a quantity of fluid contained in the fluid-inflatable chamber;
- a pressure sensor for monitoring a pressure inside the fluid-inflatable chamber; and
- a controller configured to
- implement a defined volume change procedure using the volume varying arrangement, and
- monitor an internal pressure within fluid-inflatable chamber during the volume change procedure, and generate an output based on the monitored pressure during the volume change procedure.

Embodiments of the invention are effectively configured to perform a calibration procedure based on determining in real time, during use, a relationship between volume changes in the inflation chamber and detectable internal pressure changes in the chamber. This is done by artificially changing a volume capacity inside the fluid-inflatable chamber without increasing or reducing the amount of gas in the fluid-inflatable chamber, for instance by pressing onto a distensible wall of the chamber from outside to partially compress the fluid inside, or otherwise occupying a portion of the chamber volume. This imposes a secondary (relatively small) volume change, on top of the global inflation level of the device (as part of the blood pressure measurement process), from which an (instantaneous) relationship between volume changes and pressure changes inside the chamber can be deduced.

This may then be subsequently used in determining arterial compliance.

A measurement process can be performed using the measurement device and the derived information relating fluid inflatable chamber pressure versus fluid inflatable chamber volume. In particular, sensed pressure changes inside the chamber during the measurement may be taken to be representative of arterial pressure, $P_a$. oscillations. The derived pressure-volume relationship can then be used to determine corresponding volume changes inside the chamber caused by arm (or body part) displacement, representative of arterial volume changes, $V_a$. From these two measures ($P_a$ and $V_a$ changes), arterial compliance may for example be deduced (using equation (1) set out above).

The actual deriving of arterial compliance is optional. Embodiments of the invention provide a means for calibrating the device sufficient to enable such determinations to be performed more accurately and reliably.

Preferably, the artificial volume changes induced are configured so as to generate pressure change responses which have similar characteristics to those generated in response to arterial volume changes. For example, the volume change procedure may be configured to induce oscillations in the volume (so as to resemble the oscillatory volume response to arterial pressure oscillations). The volume change procedure maybe configured to induce volume oscillations having a frequency within the same order of magnitude as a typical frequency of arterial pressure oscillations, e.g. oscillations between 1Hz and 10Hz. The volume change procedure may be configured to induce volume oscillations having an amplitude within the same order of magnitude as volume oscillations generated in response to arterial pressure oscillations. This may be empirically derived for example.

More generally, the volume change procedure may be configured based on prior knowledge of factors affecting the fluid-inflatable chamber response to arterial pulsation. For example, material viscoelastic properties of the fluid-inflatable chamber might cause artificial oscillatory volume changes of high frequency (e.g. around 50 Hz) to generate a significantly different pressure response compared to a pressure response generated by arterial volume changes (which contains frequency components in the range 1Hz - 10 Hz).

The fluid-inflatable chamber may be a distensible chamber for instance. The chamber may have one or more distensible or compliant walls. For example, the fluid-inflatable chamber may take the form of an inflatable bladder, bag, or balloon.

The volume change procedure implemented by the controller may comprise a set of one or more volume changes. The volume changes may be all in the same direction (all increases, or all decreases), or in different directions (up and down). The volume change procedure may comprise an induced oscillation in the internal chamber volume.

The fluid received by the chamber is preferably a partially compressible fluid, e.g. a gas, e.g. air.

The blood pressure measurement device is preferably an oscillometric blood pressure measurement device, configured for measuring a blood pressure of a subject based on sensing oscillations in pressure inside the cuff. It may be a sphygmomanometer in examples.

The controller may be additionally configured to control the device to perform blood pressure measurements, based on varying an applied pressure through a range of applied pressures and sensing pressure oscillations in the fluid-inflatable chamber across the range of applied pressures. This may be performed concurrently with the calibration procedure comprising performing the volume change procedure and monitoring pressure variations inside the chamber in response. Where done concurrently, signal processing (e.g. filtering) may be applied to the pressure sensor output to separate pressure signal components corresponding to arterial pressure oscillations and signal components corresponding to artificially induced chamber volume changes.

The pressure sensor monitors a fluid pressure inside the chamber. It may generate an output pressure signal.

The output generated by the controller may be a data output.

The blood pressure measurement device may be a blood pressure measurement cuff for wrapping for instance around an arm or a leg of the subject in use.

The controller may be configured to determine an internal pressure versus volume relationship for the chamber based on the monitored pressure values across the volume change procedure. Hence here, the generated output is the pressure versus volume relationship. This may be a pressure change versus volume change relationship, or for instance a rate of change of pressure with volume (or vice versa).

The pressure versus volume relationship may be a pressure-volume function.

The pressure versus volume relationship essentially provides a calibration of the blood pressure measurement device, as discussed above, from which a measure of arterial compliance can subsequently be derived.

Alternatively, according to one or more embodiments, the controller may be adapted to implement a feedback loop for controlling the volume change procedure based on the internal pressure signal, wherein the volume is controlled (for instance recurrently adjusted) so as to keep the internal pressure at some constant value. The output generated by the controller in this case may be an output representative of the required volume change to keep the pressure constant. The output may be an output signal indicative of a series of volume change values, or volume change oscillations over time.

The output may be taken as directly representative of the arterial volume oscillations.

As discussed above, according to examples, arterial volume can be derived from sensed pressure oscillations in the cuff using a known compliance of the blood pressure measurement device. The compliance may encompass for instance fluid-inflatable wall elasticity properties, as well as for instance air compression properties inside the chamber.

Hence, according to one or more embodiments, the controller may be configured to derive a measure of compliance (or elasticity) of a distensible wall of the fluid-inflatable chamber using the determined pressure versus volume relationship.

Compliance is directly related to rate of change of volume as a function of pressure. Compliance may for example be taken as equal to $dV_c/dP_c$, where $V_c$ is chamber volume, and $P_c$ is chamber pressure.

According to one or more embodiments, the controller may be configured to determine a measure representative of arterial compliance of the subject using the monitored pressure values over the implemented volume change procedure.

For example, according to at least one set of embodiments, the deriving the measure representative of arterial compliance may comprise the following steps:

- varying an inflation level of the fluid-inflatable chamber to thereby vary a pressure applied to said part of the body across a range of applied pressures; and
- at each of one or more of said range of applied pressures:

  - implementing said defined volume change procedure using the volume varying arrangement, monitoring the internal pressure within the chamber during the volume change procedure, and deriving a pressure versus volume relationship, and
  - without driving the volume varying arrangement, acquiring a pressure variation signal based on monitoring internal pressure change oscillations within the chamber using the pressure sensor; and
  - deriving a volume variation signal using the acquired pressure variation signal and the derived pressure versus volume relationship.

The pressure variation signal is acquired at a fixed configuration of the volume varying arrangement, i.e. the only changes in the volume in the chamber are those generated by arterial pressure oscillations pressing on the chamber from the subject's body part.

Following the above steps, deriving the measure representative of arterial compliance may further comprise deriving the measure of arterial compliance based on the derived volume variation signal and the acquired pressure variation signal. Arterial compliance may be taken to be equal to the rate of change of the derived chamber volume with measured chamber pressure, such that chamber pressure variations are assumed to be representative of arterial pressure and calculated chamber volume changes assumed to be representative of arterial volume changes.

According to one or more embodiments, the volume varying arrangement may be arranged to exert a controllable pressure onto a wall of the chamber to induce the controllable volume change. The pressure may be applied for example from outside the chamber, e.g. pressing onto an external bounding wall of the chamber. The pressure may be exerted onto a distensible wall of the chamber for instance.

For example, the volume varying arrangement may be adapted to exert a controllable pressure onto a distensible wall of the chamber to induce the volume change, i.e. press onto the chamber wall from the outside.

The device may be an actuator for instance, arranged to press onto the wall. This may include a motorized or mechatronic actuator.

However, in alternative examples, the volume varying arrangement may include a variable volume occupying element disposed interior of the chamber, operable to change volume, and thus to change a proportion of the chamber volume which is occupied. This provides one further means for varying an internal volume of the fluid inflatable chamber without changing an amount of fluid in the fluid-inflatable chamber.

In some examples, the volume varying arrangement may comprise a secondary fluid-inflatable chamber having a controllable inflation level. It may have a controllable internal fluid volume, and/or a controllable amount of contained fluid.

For example, the secondary fluid chamber may be arranged to exert a pressure onto the first fluid-inflatable chamber, for instance from outside the chamber, e.g. onto a wall of the chamber. It may be arranged in pressing relationship with the first fluid-inflatable chamber.

For example, the secondary fluid-inflatable chamber may be adapted in use to exert a controllable pressure onto the first fluid-inflatable chamber as a function of a fluid inflation level of the secondary chamber to thereby implement the defined volume change scheme.

In preferred examples, the secondary fluid chamber may be inflatable with a liquid, i.e. is a liquid-inflatable chamber. In this case, the secondary chamber is adapted to receive a variable quantity of liquid, e.g. water.

Liquid is preferred as the inflation fluid for the secondary chamber because liquid, e.g. water, is incompressible. This means that the outer volume occupied by the secondary chamber can be known with greater certainty, since there is no possibility that its volume will compress on contact with another body, such as the first inflatable chamber. Hence use of liquid allows a more controllable volume variation to be implemented.

Preferably, the main (first) fluid-inflatable chamber may be a gas-inflatable chamber, e.g. inflatable with air. This is because gas is a compressible fluid. This allows for the volume of the first chamber to be easily varied in certain examples by simply exerting a pressure on the chamber (e.g. the chamber wall), to thereby compress the contained gas to some extent, and this reduce its occupying volume.

According to one or more examples, the blood pressure measurement device may comprise a body-mountable cuff. The fluid-inflatable chamber may be internal to the cuff. The cuff may be for wrapping around a subject's arm in use.

The cuff may include a compliant, e.g. distensible, wall.

In the case of the above example, the secondary chamber may be in the form of a secondary cuff comprising a secondary fluid-inflatable chamber. The secondary cuff may be arranged concentrically with the body-mountable cuff comprising the first fluid-inflatable chamber.

The secondary cuff may in certain examples be arranged on a body-facing side of the body-mountable cuff, for example for applying pressure onto a body-facing side of the body-mountable cuff. The body-facing side may mean a radially interior side of the primary cuff for example.

For example, the secondary cuff may be arranged for applying pressure onto body-facing external surface of the body-mountable cuff, for example an external surface of a distensible wall of the cuff.

Examples in accordance with a further aspect of the invention provide a method of configuring a blood pressure measurement device,

the device being for mounting to a part of a body of a subject, for applying a variable pressure in use to said part of the body, and the device comprising a fluid-inflatable chamber for receiving a variable quantity of fluid for inducing said variable pressure to the part of the body in use,

and the method comprising:

- implementing a defined volume change procedure comprising inducing one or more defined changes in an internal volume of the chamber without altering a quantity of fluid contained in the chamber; and
- monitoring an internal pressure within chamber during the volume change procedure, and generating an output based on the monitored pressure during the volume change procedure.

Implementation options and details for each of the above steps may be understood and interpreted in accordance with the explanations and descriptions provided above for the apparatus aspect of the present invention (i.e. the device aspect).

Any of the examples, options or embodiment features or details described above in respect of the apparatus aspect of this invention (in respect of the blood pressure measurement device) may be applied or combined or incorporated mutatis mutandis into the present method aspect of the invention.

According to one or more embodiments, generating said output may comprise deriving an internal pressure versus volume relationship for the fluid-inflatable chamber based on said monitored pressure values across the volume change procedure.

According to one or more embodiments, the method may further comprise determining a measure representative of arterial compliance of the subject using the monitored pressure values over the implemented volume change procedure.

These and other aspects of the invention will be apparent from and elucidated with reference to the embodiment(s) described hereinafter.

BRIEF DESCRIPTION OF THE DRAWINGS

For a better understanding of the invention, and to show more clearly how it may be carried into effect, reference will now be made, by way of example only, to the accompanying drawings, in which:

Fig. 1 schematically illustrates an example blood pressure measurement device according to one or more embodiments;
Fig. 2 shows a cross-sectional plan view through a further example blood pressure measurement device in accordance with one or more embodiments;
Figs. 3 and 4 show sample pressure versus volume relationships derived according to an embodiment of the invention by means of an applied volume change procedure; and
Fig. 5 schematically illustrates an example pressure variation signal obtained according to a volume change procedure in accordance with one or more examples.

DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a blood pressure measurement device having an inflatable chamber with a variable inflation level for applying a controllable pressure to a part of a subject's body, and a volume varying arrangement for inducing an additional change in an internal volume of the chamber without altering a quantity of fluid inside the chamber. A controller is configured to execute a volume change procedure using the volume varying arrangement, wherein one or more defined volume changes are induced in the chamber, with the fluid quantity kept the same. An internal pressure change response inside the chamber is simultaneously monitored. An output is then generated based on the sensed pressure changes. In some examples, a pressure versus volume relationship for the inflatable chamber may be generated and output.

By directly measuring a pressure response to volume variations, this derived relationship can be used to directly convert measured arterial pressure oscillations into corresponding arterial volume oscillations (i.e. the volume change of the artery which caused the measured pressure change). This may allow arterial compliance to be derived solely based on oscillometric measurement signals.

As discussed above, in principle, information about the status of the arterial smooth muscle tone (or arterial compliance) can be obtained from oscillometric data. This is due to the fact that oscillometry reveals the amplitude of arterial volume oscillations under a range of transmural pressures (by artificially applying to the artery a range of applied pressures. These amplitudes depend on mechanical properties of the vessel wall. However, measuring the arterial volume pulsations via oscillometry is not straightforward. As oscillometry data is recorded by means of measuring pressure inside the inflation chamber, a translation from recorded pulses in internal chamber pressure to pulses in arterial volume is necessary. This translation may be described as chamber compliance - the relationship between a change in measurement device volume and a change in measurement device pressure.

Inflation chamber compliance is a function of the measurement cuff (or other device) volume, cuff material elastic properties, temperature, absolute cuff pressure and possibly amplitude and frequency of oscillations. Therefore, the translation from pressure pulse to volume pulse can change depending upon tightness of device wrapping about the body part, arm size, arm tissue characteristics, temperature of cuff air, inflation speed, frequency components of arterial waveform (viscoelasticity of measurement device material), time passed since previous inflation and other factors.

Embodiments of the present invention propose wherein inflation chamber compliance is additionally measured, for instance during an oscillometric measurement as an additional step in the measurement method. This may be done by means of producing controlled changes to the cuff volume, for instance during an oscillometric measurement, and analyzing the resulting changes in pressure. This technique reduces unreliability and uncertainty that presently exists regarding the pressure-volume translation, and so enables oscillometric-based arterial compliance measurements to be performed much more reliably.

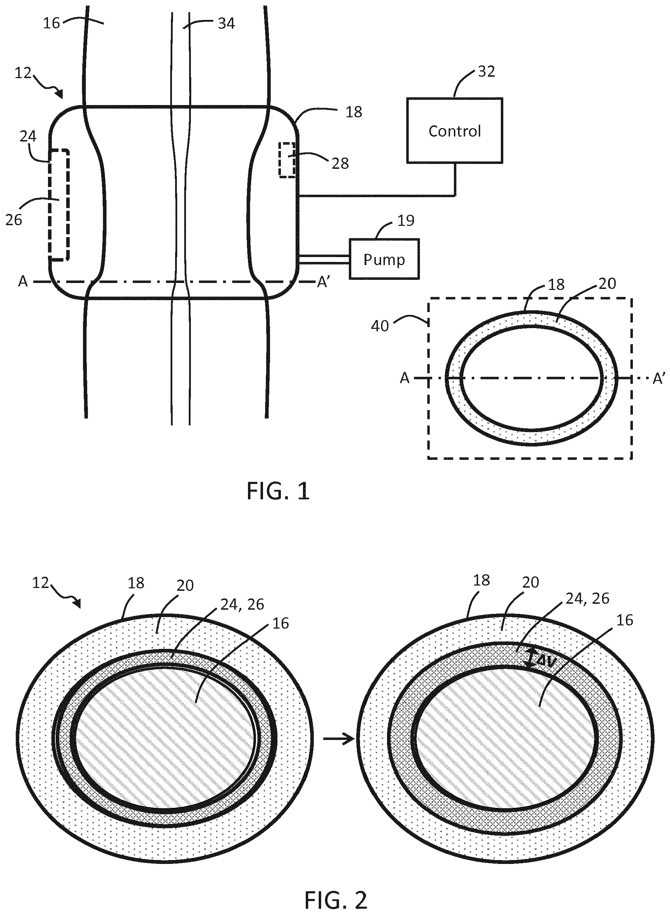

Fig. 1 shows one example blood pressure measurement device 12 in accordance with one or more embodiments.

The device 12 is for mounting to a part of the body of a subject, for applying a variable pressure in use to said part of the body. In the present case, the device takes the form of an inflatable blood pressure measurement cuff for wrapping around the arm 16 of a subject, for applying a variable pressure to the subject's arm 16. The variable pressure onto the body part induces an applied pressure to an artery 34 within the body part, for example the brachial artery.

The device includes a fluid-inflatable chamber 20 for receiving a variable quantity of fluid for inducing said variable pressure to the part 16 of the body in use. The applied pressure is controllable as a function of an inflation level of the fluid-inflatable chamber 20. In the present example, the fluid-inflatable chamber 20 is provided internal of the cuff 18. The fluid-inflatable chamber is illustrated in the dashed box 40 at the bottom-right hand-side of Fig. 1. The dashed box depicts a cross-sectional plan view through the cuff 18 along indicated cut line A-A'.

Changing the fluid quantity changes the inflation level of the fluid-inflatable chamber 20. The chamber takes the form of a distensible or expandable bladder, having distensile or compliant walls which stretch outwardly to accommodate additional fluid received into the chamber. An outer volume of the chamber thus expands as a function of contained fluid quantity.

The quantity of fluid contained in the chamber 20 is controllable such that the fluid-inflatable chamber has a controllable inflation level. For example, the device may comprise a fluid pump 19 for controlling a quantity of fluid contained in the fluid-inflatable chamber 20 of the cuff 18. The device may include a separate control unit for controlling a fluid inflation level of the cuff inflatable chamber using the pump. Alternatively the central controller 32 may perform this function.

In the present example, the fluid-inflatable chamber is an air-inflatable chamber, since air is at least partially compressible. This makes adjusting or varying a volume of the chamber without changing the fluid quantity contained therein much easier (based on simply compressing the chamber).

The blood pressure measurement device 12 according to the present example is an oscillometric blood pressure measurement device, and is configured for facilitating blood pressure measurements of a patient based on measuring pressure variations (in particular, pressure oscillations) within the internal inflation chamber 20 at different levels of overall global inflation of the cuff, i.e. different applied pressures. These pressure variations inside the inflation chamber correspond to arterial blood pressure oscillations. By measuring these oscillations at different applied pressures, the arterial pressure at different transmural pressures is obtained, allowing a full blood pressure measurement to be performed.

A pressure sensor 28 is included in the device 12 for monitoring a pressure inside the internal chamber. The sensor may for example be disposed inside the fluid-inflatable chamber 20 of the cuff 18.

A volume varying arrangement 24 is further included for inducing a defined change in an internal volume of the fluid-inflatable chamber 20 of the device 12 without altering a quantity of fluid contained in the chamber.

The device further comprises a controller 32. The controller is operatively coupled at least to the volume varying arrangement 24 and the pressure sensor 28. The controller may be further operatively coupled to the inflation control of the fluid-inflatable chamber 20 of the cuff, for example the air pump 19 in Fig. 1.

The controller is configured to in use to implement a defined volume change procedure using the volume varying arrangement 24, and to at the same time monitor an internal pressure within the chamber 20 (using the pressure sensor 28) during the volume change procedure, and generate an output based on the monitored pressure during the volume change procedure.

The output may be representative of a chamber pressure versus volume relationship. The output may be representative of a compliance or elasticity of the chamber, or of distensible wall(s) of the chamber. The output may alternatively be representative of a necessary volume change as a function of time to maintain a pressure inside the chamber constant. This will be explained in more detail to follow.

In some examples the controller 32 may also be configured to control the inflation level of the fluid-inflatable chamber 20 so as to perform blood pressure measurements in use, in addition to controlling the volume variation procedure and deriving the pressure-related output.

The volume change procedure implemented by the controller 32 may comprise one or more discrete volume changes of the inflatable chamber 20. It may comprise volume change oscillations applied atop an overall global inflation level of the inflation chamber 20.

In examples, the volume change procedure may comprise a modulation of the volume, for instance in the form of applied volume oscillations, which may be small in amplitude or magnitude in comparison with an overall global level of the chamber volume (or total chamber capacity).

Preferably, the artificial volume changes induced are configured so as to generate pressure change responses which have similar characteristics to those generated in response to arterial volume changes. For example, the volume change procedure may be configured to induce oscillations in the volume (so as to resemble the oscillatory volume response to arterial pressure oscillations). The volume change procedure maybe configured to induce volume oscillations having a frequency within the same order of magnitude as a typical frequency of arterial pressure oscillations, e.g. oscillations between 1Hz and 10Hz. The volume change procedure may be configured to induce volume oscillations having an amplitude within the same order of magnitude as volume oscillations generated in response to arterial pressure oscillations. This may be empirically derived for example.

More generally, the volume change procedure may be configured based on prior knowledge of factors affecting the fluid-inflatable chamber response to arterial pulsation. For example, material viscoelastic properties of the fluid-inflatable chamber might cause artificial oscillatory volume changes of high frequency (e.g. around 50 Hz) to generate a significantly different pressure response compared to a pressure response generated by arterial volume changes (which contains frequency components in the range 1Hz - 10 Hz). Therefore, the volume change procedure may be configured based on a pre-determined typical amplitude and/or frequency of volume changes induced by typical arterial pressure oscillations.

The volume varying arrangement 24 may be any element suitable for inducing a controllable change in the internal volume of the fluid-inflatable chamber 20 of the blood pressure measurement device 12 without altering a quantity of fluid in the chamber (e.g. the chamber of the cuff 18 in the example of Fig. 1 above).

Various options are possible for the volume varying arrangement.

According to one set of examples, the volume varying arrangement may comprise a secondary fluid-inflatable chamber 26 having a controllable inflation level. The secondary fluid-inflatable chamber maybe adapted in use to exert a controllable pressure onto the first fluid-inflatable chamber 20 as a function of a fluid inflation level of the secondary chamber to thereby implement the defined volume change scheme. The secondary chamber may be a distensible chamber, e.g. having distensible walls, meaning that the outer volume occupied by the chamber expands as more fluid is added to the chamber. It may hence take the form of an inflatable bladder.

Fig. 1 shows one example comprising a volume varying arrangement 24 taking the form of a secondary fluid-inflatable chamber 26. The secondary fluid-inflatable chamber may be operatively coupled with an inflation control element, e.g. a local fluid pump, for controlling a level of fluid comprised by the secondary fluid-inflatable chamber. The secondary chamber is arranged in this example on one side of the cuff 18, exterior of the primary fluid-inflatable chamber 20. The secondary fluid-inflatable chamber is arranged so as to exert a varying pressure onto the primary inflatable chamber 20 of the cuff as the fluid level inside the secondary chamber 26 is varied. The pressure exerted onto the primary chamber 20 is a function of the secondary chamber 26 inflation level.

As an inflation level of the secondary chamber 26 is increased, so it exerts an increasing pressure onto the primary chamber 20. The amount of fluid inside the primary chamber is not changed during this procedure. As the applied pressure by the secondary chamber 26 increases, so the primary chamber 20 becomes partially compressed. This reduces an internal volume of the primary chamber, thereby compressing the contained fluid into the smaller internal volume, thus reducing the volume of the contained fluid. As the amount of fluid stays the same, and the volume is reduced, so the pressure of the fluid increases (inversely) proportionate with the volume change (or proportionate with the volume reduction).

By monitoring the pressure change during induced volume changes, a volume-pressure relationship for the fluid-inflatable chamber 20 of the device 12 can be determined according to examples. This relationship may be referred to as a pressure-volume transfer function.

According to one or more examples, the secondary chamber 26 may be provided in the form of a secondary cuff comprising a secondary fluid-inflatable chamber. The secondary cuff may be arranged concentrically with the body-mountable cuff 18 comprising the first fluid-inflatable chamber 20.

One example of such an arrangement is illustrated schematically in Fig. 2. Fig. 2 shows a cross-sectional plan view through a blood pressure measurement device 12 across a plane perpendicular to a longitudinal axis of the device (parallel with radial and circumferential axes).

The device 12 comprises a primary body-mountable cuff 18, having an integrated air-inflatable chamber 20 internal to the cuff. In this example, the cuff is for wrapping around a subject's arm 16. Fig. 2 illustrates the device in use, fitted around a subject's arm 16.

The device 12 comprises a volume varying arrangement 24 in the form of a secondary body-mountable cuff, arranged concentrically with the primary cuff 18. The secondary cuff is arranged to make contact with a radially interior side of the primary cuff, i.e. a body facing side. The secondary cuff may have a smaller total volume capacity than the primary cuff. It may have a smaller longitudinal length than the primary cuff, so as to only extend along a portion of the arm length of the primary cuff in use.

As in the example of Fig. 1, an inflation control element, such as a fluid pump, may be provided operatively coupled with the secondary cuff, for controlling an amount of fluid contained in the secondary cuff, and thus an inflation level.

Fig. 2 schematically illustrates implementation of an example volume change procedure using the secondary fluid-inflatable cuff 24 as a volume varying arrangement.

Fig. 2 (left) shows a cross-section through the device in an initial state, before a volume change has been implemented.

Fig. 2 (right) shows the device after the secondary (inner) cuff chamber 26 has been partially inflated. The inflation increases an outer volume of the secondary cuff. This exerts a pressure on the primary cuff in a radial outward direction. So long as a circumference of the primary cuff 18 is not varied (i.e. the cuff remains tightly wrapped around the subject's arm 16), this results in a compression of the fluid within the primary chamber 20, and a reduction of the interior volume of the primary chamber 20.

The resulting pressure change inside the primary chamber 20 as this volume change is implemented is sensed using a fluid pressure sensor 28 disposed inside the primary chamber (not shown in Fig. 2).

The complete volume change procedure may involve a series of multiple such volume variations, or a single continuous volume change may be performed. The pressure response inside the primary chamber 20 is monitored throughout to derive a pressure response signal as a function of induced volume change.

Although in the above examples, the volume varying arrangement is shown as comprising a secondary fluid-inflatable chamber, other options are possible.

According to one set of examples for instance, the volume varying arrangement may comprise a variable volume occupying element disposed interior of the chamber 20, operable to change volume, and thus to change a proportion of the inflatable chamber 20 interior volume which it occupies. This variable volume element may be an inflatable element, such as an inflatable bladder. It may be provided with a pneumatically controllable volume (variable for instance anywhere between a fully inflated state and a fully deflated state).

According to further examples, the volume varying arrangement may comprise a mechanical or mechatronic device, such as actuator, placed exterior of the inflatable chamber, and operable to exert a controllable pressure onto the chamber (e.g. onto a bounding wall of the chamber). The movement of the device pressing against the exterior of the chamber wall here may provide the source of controlled volume changes. The mechanical element may for example take the form of a spring or a coil, or a motor, or any other form of actuator.

According to any example, a dedicated local control element, such as a microcontroller may be provided for controlling the movement of the volume varying arrangement 24 in implementing the volume change procedure. Alternatively, the volume varying arrangement may be directly controlled by the central controller 32 of the blood pressure measurement device 12.

According to one or more possible embodiments, there may be provided a volume varying arrangement configured to automatically adjust a level of volume change induced within the fluid inflatable chamber based on a pressure inside the fluid-inflatable chamber. For example, the volume varying arrangement may comprise a component situated inside the fluid inflatable chamber formed of a pressure-responsive material. The component may be configured to deform in response to pressure changes, for instance by an amount which varies as a function of the pressure inside the chamber. The component may be a wall of a volume-occupying element within the fluid-inflatable chamber, configured to collapse by a defined extent as a function of pressure inside the chamber to thereby induce a defined change response in the volume being occupied by the element as a function of pressure.

This allows for instance the volume change procedure to be driven intrinsically by induced pressure changes in the chamber caused by inflation and deflation of the chamber during use, for instance during blood pressure measurements.

According to any example, the volume change procedure may comprise a series of volume change pulses. This may comprise a series of induced step changes (e.g. reductions) in the volume of the primary fluid-inflatable chamber 20, of a defined volume change size, and each lasting a defined duration.

The controller 32 implements a procedure in use which allows the pressure change response of the cuff 18 internal fluid chamber 20 to defined volume changes to be measured. A compliance of the inflatable chamber may be derived.

This information in turn may allow arterial volume oscillations of the artery 34 to be derived from measured pressure oscillations inside the cuff when performing blood pressure measurements. This is because the derived pressure-volume relationship for the chamber allows subsequently measured arterial pressure oscillations during a blood pressure measurement to be converted into arterial volume oscillations.

Optionally, from the thus derived arterial volume oscillations and the corresponding measured pressure oscillations, a measure of arterial compliance can be derived.

Various approaches can be implemented in different examples for deriving relevant information from the measured pressure changes during performance of the volume varying procedure using the volume varying arrangement 24. The particular output generated can vary in addition to the procedure by which it is calculated. All examples share in common however that an internal pressure response within the primary fluid-inflatable chamber of the device is monitored as one or more volume variations are induced in the primary chamber internal volume.

According to one set of advantageous examples, the volume variation procedure maybe implemented concurrently with performing blood pressure measurement of a patient using the inflatable cuff 18 of the blood pressure measurement device. This may comprise processing a measured pressure variation signal throughout the procedure with a signal extraction or modulation scheme to separate or extract signals corresponding to oscillations of the arterial wall and corresponding to the controlled volume changes.

To enable better understanding of embodiments of the invention, the procedure for performing a standard blood pressure measurement will first be described.

In a standard blood pressure measurement, the measurement device is first attached to an appropriate part of the body, arranged for applying a variable pressure to that part, and in particular to an artery comprised in that part. For examples, a blood pressure measurement cuff may be wrapped around a subject's arm.

A blood pressure measurement procedure then comprises varying an inflation level of the fluid-inflatable chamber of the device across a range of applied pressures to thereby vary a pressure applied to the part of the body to which the device is mounted. For example, the chamber 20 may be gradually inflated so as to increase a pressure applied to the artery through a range of increasing pressures. Alternatively, the chamber 20 may in use be inflated to fully occlude the artery in question before the applied pressure is gradually reduced through a range of decreasing applied pressures.

At each of one or more of the applied pressures, an internal chamber pressure is monitored and a pressure signal acquired indicative of pressure oscillations inside the chamber. For a given (fixed) chamber inflation, such oscillations can be taken to be representative of variations in pressure applied to the device by the underlying artery as it pulses with blood. These oscillations may hence be taken as representative of oscillations in arterial pressure of the underlying artery.

According to embodiments of the present invention, this blood pressure measurement procedure may be augmented with a procedure for determining a pressure versus volume relationship for the chamber, based on performing a volume variation procedure.

According to one or more examples, this may comprise implementing, at each of one or more of said range of applied pressures, a defined volume change procedure using the volume varying arrangement, monitoring the internal pressure within the chamber during the volume change procedure, and deriving a pressure versus volume relationship.

As noted above, this may require separating or extracting from the measured pressure variation signal (from the pressure sensor) separate components corresponding to the arterial pressure oscillations and to the artificially induced volume variation components. They may be distinguished or separated based on differing frequency for example.

An example of this procedure has been implemented using a blood pressure measurement device comprising a primary inflatable cuff 18, and comprising a volume varying arrangement in the form of a secondary inflatable cuff, as in the example of Fig. 2 above.

The secondary cuff 24 used is a liquid-inflatable cuff. The secondary cuff 24 was positioned at a skin-facing surface of the primary cuff 18, i.e. positioned in use between the subject's arm 16 and the primary cuff 18.

As the primary cuff 18 is inflated from atmospheric pressures to above systolic pressure, water in the secondary cuff 24 is alternately added and removed, at regular intervals. These secondary cuff inflation level changes vary a pressure applied onto the primary cuff by the secondary cuff to thereby induce defined volume changes in the internal volume of the primary cuff 18 inflatable chamber 20.

In the example implementation, a series of defined volume changes of 3 ml were induced in the primary inflatable chamber 20 internal volume by means of the secondary inflatable cuff 24.

These well-defined 3 ml changes in the primary chamber 20 volume create corresponding pressure changes within the primary chamber. A pressure-volume transfer function may be derived by associating the induced 3 ml volume pulses to the corresponding sensed pressure changes. This transfer function enables an estimation of artery dilatation (arterial volume change) to be subsequently determined, under the entire range of transmural pressures (i.e. under the full range of applied pressures exerted by the primary blood pressure measurement cuff to the arm 16). Due to the independently derived cuff volume-pressure relationship, this can be calculated independently of tightness of cuff wrapping, arm size, body/environment temperature, cuff inflation speed or any other variable.

Preferably, the secondary fluid-inflatable chamber 26 of the secondary cuff 24 is inflated with liquid, e.g. water, instead of air, so that the volume of this secondary chamber is incompressible. This ensures that the magnitude of volume changes induced in the primary chamber by the secondary chamber can be more reliably known, since the volume of the secondary chamber 26 does not compress when pressed against the primary chamber (the volume of the secondary chamber does not change with pressure).

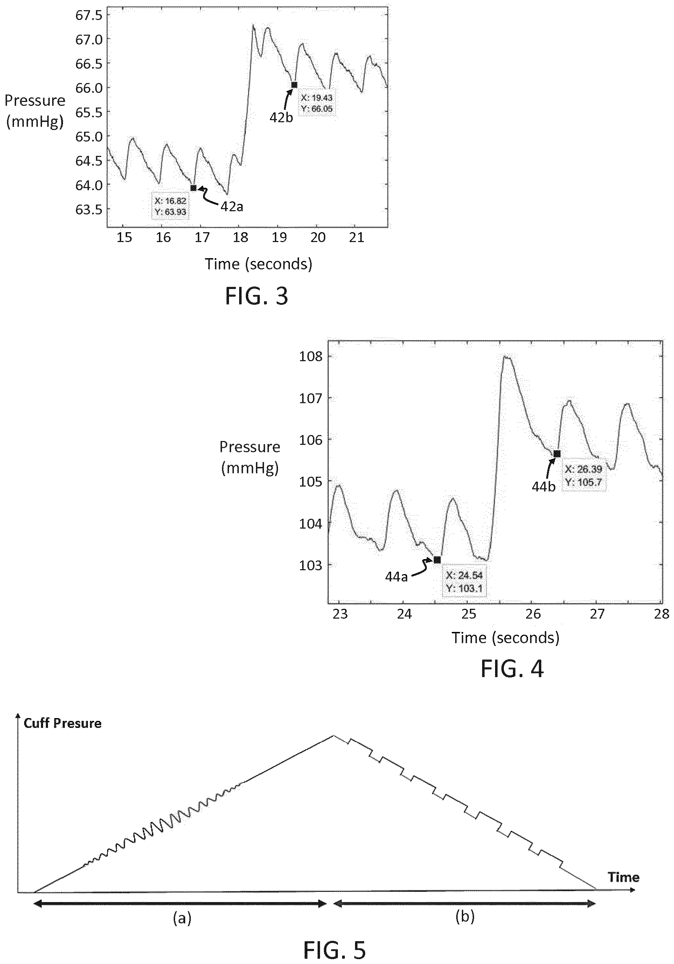

Figs. 3 and 4 show example measured pressure response signals (y-axis; mmHg) inside the primary fluid-inflatable chamber as a sample volume change procedure is implemented using the secondary inflatable chamber 26 of the secondary cuff 24. The pressure response signal in each graph is shown as a function of time (x-axis, seconds).

The volume change procedure was implemented concurrently with implementation of a blood pressure measurement. Accordingly, the acquired pressure signal includes signal components corresponding both to arterial pressure oscillations (pressing onto the chamber and causing regular peaks in the measured pressure) and deliberately induced volume changes using the secondary inflatable chamber 26. The arterial pressure pulses are visible as the relative higher frequency oscillations, with a relative small amplitude. The larger amplitude change in each graph corresponds to the artificially induced volume changes, generated by introducing the 3 ml injections of water into the secondary inflatable chamber of the secondary cuff.

A measure of compliance or elasticity of the primary inflatable chamber 20 (or of distensible walls of the chamber) maybe derived from the measured pressure response signals, based on a known frequency, or known time points of the controlled volume changes. This may allow the arterial pressure oscillation signal components and the induced volume variation signal components to be separated or distinguished from one another.

For example, pressure may be measured before and after each volume change time point, to thereby determine the pressure change caused by the respective volume change. In this way, pressure change response as a function of volume change can be determined.

The pressure before and after a given volume change may be measured at respective fiducial points 42a, 42b, 44a, 44b on the high frequency signal, as shown in Figs. 3 and 4. For the particular cuff wrapping tightness of the present examples, and at an absolute (global) cuff inflation chamber 20 pressure of 64 mmHg, the pressure change caused by an induced 3 ml volume change is 2.09 mmHg (difference in pressure between point 42a and 42b). At absolute (global) cuff inflation chamber 20 pressure of 103 mmHg, the pressure change caused by a 3 ml volume change is 2.5 mmHg (difference in pressure between point 44a and 44b). This cuff compliance information enables the deriving of arterial volume oscillations from measured arterial pressure oscillations.

The induced volume change pulses may be controlled to occur at regular intervals, and/or their timings (and amplitude) may be configured to account for breathing artefacts in the arterial pulse signal, inflation speed of the cuff, and/or any other factors, such as other factors related to the background state of the system.

According to certain examples, the volume change procedure (e.g. volume change pulses) may be implemented recurrently or continuously throughout performance of a blood pressure measurement. This ensures that at each of the set of applied pressures to the body part (at each of which arterial pressure is measured), an up-to-date and accurate conversion function between sensed pressure oscillations and corresponding arterial volume oscillations is known.

Where the procedure for deriving the volume-pressure relationship (using the volume change procedure) is performed concurrently with blood pressure measurement, signal processing may be applied to the acquired pressure response signal inside the primary inflatable chamber in order to separate or extract the arterial pressure oscillation components from the induced volume variation components.

This signal processing may comprise a simple frequency-based filtering process, for instance application of appropriate high or low pass filters: a high pass filter for extracting the arterial signal components, a low pass filter for extracting the deliberate induced volume change components.

The frequency of the controlled volume change pulses may be chosen to make such a separation process easier, i.e. selecting a frequency significantly smaller than the arterial pressure pulse frequency. The viscoelastic behavior of the primary inflatable chamber 20 may be taken into account in the choice of frequency of the controlled volume change pulses. For example, some frequencies may be effectively filtered out due to damping caused by the elastic response behavior of the elastic cuff material.

For example, given a step volume pulse (an increase) which causes an increase in cuff pressure, the cuff material may take a short time delay before distending (e.g. related to the viscosity of the fluid). If instead of the step pulse, fast-changing square pulses are applied (e.g. at 50 Hz), the material has no time to respond. As a result, the recorded pressure change response as a function of the volume change would be inaccurate. If this erroneous pressure-volume relationship were then applied to deduce the volume changes occurring in response to measured arterial pressure oscillations of frequency 1 Hz, the calculation result might be incorrect. This is because at a frequency of 1 Hz the material might have time to distend between oscillations (i.e. the cuff has a different pressure-volume relationship at 1 Hz compared to 50 Hz).

A further approach to separating the signal components may comprise temporally decoupling the measurements of arterial pulsations from the measurements of controlled volume changes. In particular, arterial pressure oscillations may be measured across one defined time period and the pressure change responses to induced volume changes in the inflatable chamber 20 across a different defined time period. For example, measurement of arterial pulses might be performed only during the global inflation of the primary inflatable chamber 20 of the primary cuff 18 and measurement of cuff compliance (i.e. pressure versus volume change relationship) during global deflation.

This is illustrated schematically in Fig. 5 for instance, which shows a sample measured internal pressure signal from inside the primary inflatable chamber. Over time period (a), the primary chamber is being globally inflated (to apply an increasing pressure to the subject's body part 16), and over time period (b), the pressure is being globally deflated (to gradually reduce the applied pressure). The arterial oscillations can be seen as small oscillations superposed on the continuous increase in cuff pressure (time period (a)). The induced volume change pulses can be seen as square-shaped pulses superposed atop the continuous decrease in cuff pressure (time period (b)).

Since the controlled volume changes are controlled to occur only during the deflation phase (period (b)), this enables the arterial pulsations to be recorded during time period (a) without interference.

In this case, the signal processing is simplified, since signal extraction is only required for elimination of arterial pulses during the deflation phase (period (b)). A frequency of these oscillations may already have been detected in period (a). This detected frequency may then in some examples be used to configure a frequency filter to be applied to the pressure signal in period (b) (to filter out the arterial signal oscillations).

According to a further example, a separate pulse measuring element may be provided for measuring an arterial pulse signal, e.g. a PPG sensor, a volume clamp arrangement or any other pulse sensor. The pulse frequency sensed using this separate pulse sensor may be used to inform a signal extraction or filtering element. The filter frequency of a signal filter may be set based on the measured pulse frequency, either so as to remove arterial oscillations or to extract the arterial oscillations.

According to advantageous examples, the controller 32 may be further configured to derive a measure indicative of arterial compliance based on a derived pressure-volume relationship. As noted above, arterial compliance can be defined by the expression

$$C_{art}\left(P_{tm}(t)\right) = \frac{dV_{art}\left(t\right)}{dP_{art}(t)} \qquad (1)$$

where $V_{art}$ is arterial volume, $t$ is time, $P_{art}$ is arterial blood pressure and $P_{tm}$ is transmural pressure (i.e. the net pressure across the artery wall). Transmural pressure, $P_{tm}$ is defined as the arterial blood pressure, $P_{art}$, minus any applied pressure from the outside onto the artery wall, i.e. artery internal pressure minus externally applied pressure.

A measure of arterial compliance may hence be derived by converting the sensed arterial pressure oscillations during the blood pressure measurement procedure described above into a signal representative of arterial volume oscillations using the derived pressure-volume relationship for the fluid-inflatable chamber 20.

In brief, deriving the measure representative of arterial compliance may hence comprise the following steps:

- varying an inflation level of the fluid-inflatable chamber for thereby varying a pressure applied to said part of the body across a range of applied pressures; and
- at each of one or more of said range of applied pressures:

  - implementing a defined volume change procedure using the volume varying arrangement, monitoring the internal pressure within the chamber during the volume change procedure, and deriving a pressure versus volume relationship, and
  - without varying the inflatable chamber volume, acquiring a pressure variation signal based on monitoring internal pressure change oscillations within the chamber using the pressure sensor; and
  - deriving a volume variation signal using the acquired pressure variation signal and the derived pressure versus volume relationship.

The pressure variation signal acquired with the chamber 20 volume held constant may be taken to be representative of arterial pressure variations (e.g. oscillations).

The derived volume variation signal may be taken to be representative of arterial volume variations (e.g. oscillations).

As in above examples, the volume change procedure may comprise controlling the volume varying arrangement to induce a set of one or more volume changes in the internal volume of the primary fluid-inflatable chamber 20 of the blood pressure measurement device.

The deriving of the measure representative of arterial compliance may further comprise deriving a measure of arterial compliance based on the derived volume variation signal and the acquired pressure variation signal. In particular, this can be derived using equation (1) above, by calculating the rate of change of the volume variation signal with the pressure variation signal. This may comprise dividing values of the volume variation signal by values of the pressure variation signal.

According to one or more examples, the determined pressure versus volume relationship may correspond to a measure of compliance of a distensible wall of the fluid-inflatable chamber 20. Compliance may correspond for instance to an elasticity. It may in simple cases correspond directly to a rate of change of primary inflation chamber 20 volume with internal pressure. The compliance of the measurement device inflatable chamber 20 can be used to directly convert the sensed pressure variation signal into a signal indicative of volume variations of blood within the artery at each of the range of different applied pressures.

The above represents just one example means for deriving a measure representative of arterial compliance based on monitored internal chamber 20 pressure values over the volume change procedure.

For example, according to one or more alternative examples, the controller may be adapted to implement a feedback loop for controlling the volume change procedure during arterial pressure measurement, based on the sensed arterial pressure signal. In particular, the induced volume change may be recurrently varied in response to the measured pressure signal so as to keep the internal pressure at some defined constant value. An output may be generated representative of the required volume change over the whole or a part of the blood pressure measurement to keep the pressure constant. The output may be an output signal indicative of a series of volume change values, or volume change oscillations over time.

The output in this case may be taken as directly representative of the arterial volume oscillations.

This example may potentially allow for a more precise measurement of arterial volume changes based on sensed arterial pressure changes.

This approach, in common with other example approaches of the invention, is based on implementing a deliberate set of one or more changes of the internal inflation chamber volume, and on monitoring pressure values inside the chamber responsive to these volume changes. The approach differs in that the magnitudes of the volume changes are dynamically determined in part based on the real-time result of the measured pressure-change response.

This approach may require a relatively fast response system compared to other example approaches, necessitating a processor with sufficient processing power.

As discussed above, embodiments make use of a controller 32. The controller can be implemented in numerous ways, with software and/or hardware, to perform the various functions required. A processor is one example of a controller which employs one or more microprocessors that maybe programmed using software (e.g., microcode) to perform the required functions. A controller may however be implemented with or without employing a processor, and also may be implemented as a combination of dedicated hardware to perform some functions and a processor (e.g., one or more programmed microprocessors and associated circuitry) to perform other functions.

Examples of controller components that may be employed in various embodiments of the present disclosure include, but are not limited to, conventional microprocessors, application specific integrated circuits (ASICs), and field-programmable gate arrays (FPGAs).

In various implementations, a processor or controller may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor or controller.

Examples in accordance with a further aspect of the invention provide a method of configuring a blood pressure measurement device 12,

the device 12 being for mounting to a part 16 of a body of a subject, for applying a variable pressure in use to said part of the body, and the device comprising a fluid-inflatable chamber 20 for receiving a variable quantity of fluid for inducing said variable pressure to the part of the body in use,

and the method comprising:

- implementing a defined volume change procedure comprising inducing one or more defined changes in an internal volume of the chamber without altering a quantity of fluid contained in the chamber; and
- monitoring an internal pressure within chamber during the volume change procedure, and generating an output based on the monitored pressure during the volume change procedure.

Implementation options and details for each of the above steps may be understood and interpreted in accordance with the explanations and descriptions provided above for the apparatus aspect of the present invention (i.e. the measurement device 12 aspect).

Any of the examples, options or embodiment features or details described above in respect of the apparatus aspect of this invention (in respect of the measurement device 12) may be applied or combined or incorporated mutatis mutandis into the present method aspect of the invention.

For example, according to one or more embodiments, generating said output may comprise deriving an internal pressure versus volume relationship for the fluid-inflatable chamber based on said monitored pressure values across the volume change procedure.

Additionally or alternative, according to one or more embodiments, the method may further comprise determining a measure representative of arterial compliance of the subject using the monitored pressure values over the implemented volume change procedure.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measures cannot be used to advantage. If a computer program is discussed above, it may be stored/distributed on a suitable medium, such as an optical storage medium or a solid-state medium supplied together with or as part of other hardware, but may also be distributed in other forms, such as via the Internet or other wired or wireless telecommunication systems. If the term "adapted to" is used in the claims or description, it is noted the term "adapted to" is intended to be equivalent to the term "configured to". Any reference signs in the claims should not be construed as limiting the scope.

**Claims**

1. A blood pressure measurement device (12) for mounting to a part of the body (16) of a subject, for applying a variable pressure in use to said part of the body, the device comprising:

   - a fluid-inflatable chamber (20) having a controllable inflation level for inducing said variable pressure to the part of the body in use;
   - a volume varying arrangement (24) for inducing a defined change in an internal volume of the fluid-inflatable chamber without altering a quantity of fluid contained in the fluid-inflatable chamber;
   a pressure sensor (28) for monitoring a pressure inside the fluid-inflatable chamber; and
   a controller (32) configured to

      - implement a defined volume change procedure using the volume varying arrangement (24), and
      - monitor an internal pressure within the fluid-inflatable chamber (20) during the volume change procedure, and generate an output based on the monitored pressure during the volume change procedure.

2. The blood pressure measurement (12) device as claimed in claim 1, wherein the controller (32) is configured to determine an internal pressure versus volume relationship for the fluid-inflatable chamber (20) based on the monitored pressure values across the volume change procedure.

3. The blood pressure measurement device (12) as claimed in claim 2, wherein the controller (32) is configured to derive a measure of compliance of a distensible wall of the fluid-inflatable chamber (20) using the determined pressure versus volume relationship.

4. The blood pressure measurement device (12) as claimed in any of claims 1-3, wherein the volume change procedure comprises inducing oscillatory changes in the internal volume of the fluid-inflatable chamber, and preferably wherein the oscillatory volume changes have a frequency between 1Hz and 10 Hz.

5. The blood pressure measurement device (12) as claimed in any of claims 1-4, wherein the controller (32) is configured to determine a measure representative of arterial compliance of the subject using the generated output.

6. The blood pressure measurement device (12) as claimed in claim 5, wherein the deriving the measure representative of arterial compliance comprises:

   - varying an inflation level of the fluid-inflatable chamber (20) for thereby varying a pressure applied to said part (16) of the body across a range of applied pressures; and
   - at each of one or more of said range of applied pressures:

      - implementing said defined volume change procedure using the volume varying arrangement (24), monitoring the internal pressure within the fluid-inflatable chamber (20) during the volume change procedure, and deriving a pressure versus volume relationship, and
      - without driving the volume varying arrangement, acquiring a pressure variation signal based on monitoring internal pressure change oscillations within the fluid-inflatable chamber using the pressure sensor; and
      - deriving a volume variation signal using the acquired pressure variation signal and the derived pressure versus volume relationship.

7. The blood pressure measurement device (12) a claimed in claim 6, wherein the deriving the measure representative of arterial compliance further comprises deriving a measure of arterial compliance based on the derived volume variation signal and the acquired pressure variation signal.

8. The blood pressure measurement device (12) as claimed in any of claims 1-7, wherein the volume varying arrangement (24) is arranged to exert a controllable pressure onto a wall of the fluid-inflatable chamber (20) to induce the controllable volume change.

9. The blood pressure measurement device (12) as claimed in any of claims 1-8, wherein the volume varying arrangement (24) comprises a secondary fluid-inflatable chamber (26) having a controllable inflation level.

10. The blood pressure measurement device (12) as claimed in claim 9, wherein the secondary fluid-inflatable chamber (26) is adapted in use to exert a controllable pressure onto the first fluid-inflatable chamber (20) as a function of a fluid inflation level of the secondary chamber (26) to thereby implement the defined volume change procedure.

11. The blood pressure measurement device (12) as claimed in claim 9 or 10, wherein the secondary fluid-inflatable chamber (26) is a liquid-inflatable chamber.

12. The blood pressure measurement device (12) as claimed in any of claims 1-11, wherein the blood pressure measurement device comprises a body-mountable cuff (18), and the fluid-inflatable chamber (20) is internal to the cuff, and preferably wherein the fluid-inflatable chamber (20) is an air-inflatable chamber.

13. The blood pressure measurement device (12) as claimed in claim 11 and claim 1, wherein
the secondary fluid-inflatable chamber (26) is provided by a secondary cuff comprising the secondary fluid-inflatable chamber, the secondary cuff being arranged concentrically with the body-mountable cuff comprising the first fluid-inflatable chamber.

14. A method of configuring a blood pressure measurement device (12),
the device being for mounting to a part (16) of a body of a subject, for applying a variable pressure in use to said part of the body, and the device comprising a fluid-inflatable chamber (20) for receiving a variable quantity of fluid for inducing said variable pressure to the part of the body in use,
and the method comprising:

- implementing a defined volume change procedure comprising inducing one or more defined changes in an internal volume of the fluid-inflatable chamber (20) without altering a quantity of fluid contained in the fluid-inflatable chamber; and
- monitoring an internal pressure within fluid inflatable chamber (20) during the volume change procedure, and generating an output based on the monitored pressure during the volume change procedure.

15. The method as claimed in claim 14, wherein generating said output comprises deriving an internal pressure versus volume relationship for the fluid-inflatable chamber (20) based on said monitored pressure values across the volume change procedure.

FIG. 1

FIG. 2

FIG. 3

FIG. 4

FIG. 5

Europäisches
Patentamt

European
Patent Office

Office européen
des brevets

**EUROPEAN SEARCH REPORT**

Application Number

EP 19 16 1194

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 6 027 452 A (FLAHERTY BRYAN PATRICK [US] ET AL) 22 February 2000 (2000-02-22) * abstract * * column 6, line 40 - line 51 * * column 9, line 37 - line 62 * * column 10, line 37 - column 12, line 31 * * column 10, line 53 - line 57 * * column 11, line 3 - line 5 * * column 11, line 20 - line 24 * * figures 1,8,9,13-16 * | 1-8,12, 14,15 | INV. A61B5/021 A61B5/022 A61B5/00 A61B5/02 |
| X | US 2009/312651 A1 (SANO YOSHIHIKO [JP] ET AL) 17 December 2009 (2009-12-17) * abstract * * claim 1 * | 1,9-11, 13 | |
| A | WO 2008/121454 A1 (KAZ INC [US]; FRADEN JACOB [US]) 9 October 2008 (2008-10-09) * abstract * * figures 2,3,4 * | 1-15 | |
| | | | TECHNICAL FIELDS SEARCHED (IPC)<br><br>A61B |

The present search report has been drawn up for all claims

| Place of search | Date of completion of the search | Examiner |
|---|---|---|
| The Hague | 25 April 2019 | Delval, Christophe |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 19 16 1194

This annex lists the patent family members relating to the patent documents cited in the above-mentioned European search report.
The members are as contained in the European Patent Office EDP file on
The European Patent Office is in no way liable for these particulars which are merely given for the purpose of information.

25-04-2019

| Patent document cited in search report | | Publication date | Patent family member(s) | | Publication date |
|---|---|---|---|---|---|
| US 6027452 | A | 22-02-2000 | CA | 2258263 A1 | 31-12-1997 |
| | | | EP | 0955868 A1 | 17-11-1999 |
| | | | EP | 1743572 A1 | 17-01-2007 |
| | | | JP | 3957758 B2 | 15-08-2007 |
| | | | JP | 2000512875 A | 03-10-2000 |
| | | | US | 6027452 A | 22-02-2000 |
| | | | US | 2002099296 A1 | 25-07-2002 |
| | | | US | 2004077956 A1 | 22-04-2004 |
| | | | US | 2006004293 A1 | 05-01-2006 |
| | | | US | 2006206030 A1 | 14-09-2006 |
| | | | US | 2010056930 A1 | 04-03-2010 |
| | | | US | 2011263990 A1 | 27-10-2011 |
| | | | WO | 9749328 A1 | 31-12-1997 |
| US 2009312651 | A1 | 17-12-2009 | CN | 101340845 A | 07-01-2009 |
| | | | EP | 1967133 A1 | 10-09-2008 |
| | | | JP | 4470876 B2 | 02-06-2010 |
| | | | JP | 2007167171 A | 05-07-2007 |
| | | | KR | 20080075021 A | 13-08-2008 |
| | | | TW | I379661 B | 21-12-2012 |
| | | | US | 2009312651 A1 | 17-12-2009 |
| | | | WO | 2007072647 A1 | 28-06-2007 |
| WO 2008121454 | A1 | 09-10-2008 | CN | 101730502 A | 09-06-2010 |
| | | | EP | 2139387 A1 | 06-01-2010 |
| | | | JP | 2010522610 A | 08-07-2010 |
| | | | KR | 20100027093 A | 10-03-2010 |
| | | | TW | 200843697 A | 16-11-2008 |
| | | | US | 2010106029 A1 | 29-04-2010 |
| | | | WO | 2008121454 A1 | 09-10-2008 |

EPO FORM P0459

For more details about this annex : see Official Journal of the European Patent Office, No. 12/82